# EUROPEAN PATENT APPLICATION

(11) **EP 1 847 288 A1**
(43) Date of publication of application: **24.10.2007**
(21) Application number: 06713095.5
(22) Date of filing: 06.02.2006
(51) Int. Cl.: A61M 25/00

(54) **MEDICAL CATHETER TUBE AND METHOD OF PRODUCING THE SAME**

(30) Priority: 10.02.2005 JP 2005034376
(71) Applicant: Kaneka Corporation, Kita-ku Osaka-shi, Osaka 530-8288 (JP); Kaneka Medix Corporation, Osaka-shi, Osaka 530-8288 (JP)
(72) Inventor: MURATA, Takahiro, -shi, Osaka, 5660072 (JP); MIHAYASHI, Tsuyoshi, -shi, Osaka, 5660072 (JP); OGAWA, Atsushi c/o KANEKA MEDIX CORPORATION 225, Ashigarakami-gun, Kanagawa, 258-0113 (JP); KIKUCHI, Takeshi c/o KANEKA MEDIX CORPORATION, Ashigarakami-gun, Kanagawa, 258-0113 (JP)
(74) Representative: Gille Hrabal Struck Neidlein Prop Roos
(86) International application number: PCT/JP2006/301952
(87) International publication number: WO 2006/085498

(57) **Abstract**

The medical catheter tube of the present invention integrally has an inner layer tube; reinforcement material layers formed by placing element wires on the inner layer tube; a marker; and an outer layer tube. The element wires, which are first and the other element wires, for forming the reinforcement material layers are synthetic resin element wires and/or metallic element wires, the first element wires are placed in the axis direction of the catheter to form the first reinforcement material layer, and the other element wires are wound in a coil form on the first reinforcement material layer, in the circumferential direction of the catheter, to cover the first reinforcement material layer. The marker is flexible to deformation. Because of the presence of the reinforcement material layer and the outer layer tube, flexural rigidity from a base section to a head section is reduced in a stepped or continuous manner.

## Description

### TECHNICAL FIELD

The present invention relates to a medical catheter tube superior in flexibility, positioning efficiency, torque-transmitting efficiency, kink resistance, and pressure resistance that has a higher degree of latitude in adjusting the inclination of rigidity and flexibility and the rigidity/flexibility balance according to access route and a method of producing the same.
In particular, the present invention relates to a medical catheter, of which the distal region is superior in X-ray radiopaque and also in flexibility, and that is resistant to elongation during repeated insertion and withdrawal by surgeon and deterioration of the accuracy of catheter position, and a method of producing the same.

### BACKGROUND ART

Catheter tubes are hollow medical devices that are used as inserted in the cavity, duct, blood vessel, and others in the body, specifically for selective injection of angiographic agent or others, removal of blood clot, recirculation of clogged vessel, vasodilation, and the like, and are normally tube-shaped. Such a catheter demands superior usability allowing rapid, accurate, and selective insertion thereof, for example, into thin complicated-patterned blood vessel.

More specifically as for operatibility, such a catheter tube demands favorable positioning efficiency allowing transmission of the surgeon's operation, such as insertion and withdrawal thereof into or out of the blood vessel, from the proximal region to the distal region and favorable pressure resistance, for example, when a drug solution flows inside. The catheter should also be resistant to elongation, for more accurate positional control. It also demand favorable torque-transmitting efficiency allowing reliable transmission of the torque applied in the proximal region of catheter tube and favorable insertion efficiency allowing transmission of the surgeon's force pushing the catheter tube into the blood vessel from the proximal end to the distal end. It also demand favorable guide wire compatibility, i.e., lubricity of the internal surface of the catheter, allowing insertion and withdrawal of the catheter tube along the guide wire inserted into the blood vessel in the complicated shape smoothly without damaging the blood vessel internal wall or the like, and favorable affinity to blood and organs on the external surface of the catheter. In addition, it also demands favorable kink resistance prohibiting folding of the catheter tube in the curved or bent area of blood-vessel when the distal end of catheter tube reaches a desired position and then the guide wire is removed, and favorable distal-region flexibility keeping the shape favorable for the blood vessel without damaging it.

It is generally known that a structure having a relatively rigid proximal region and a distal region gradually becoming more flexible is favorable for giving properties satisfying the requirements above.
To obtain a catheter tube having such properties described above, known is a method of forming a catheter tube by winding an internal-layer tube with a strand in the coil shape as a reinforcing-material layer or covering the internal-layer tube with an external layer of braid.

As the catheter tube having wire wound around an internal layer tube as a reinforcing-material layer, Patent Document 1 discloses a catheter tube including a catheter main body having a region where flexible internal and external tubes are connected to each other via a reinforcing-material layer, wherein the reinforcing-material layer is formed by winding a strand in a lattice shape, and the catheter main body has regions higher and lower in flexural rigidity along its the axial direction because the inclination angle of the strand to the axis of the catheter main body or the distance between the strand lattice points in the axial direction of the catheter main body varies continuously or stepwise.

However, although it is possible to form a rigid proximal region and a flexible distal region on the catheter tube, the catheter tube is not aimed at raising the degree of latitude in controlling the rigidity/flexible inclination and adjusting the rigidity/flexible balance of catheter tube according to access route. In addition, there was no specific description on a marker having X-ray radiopaque, and thus, it is not aimed at making the catheter distal region highly flexible and ensuring its X-ray radiopaque simultaneously. Moreover, no attention is give to the elongation during repeated insertion and withdrawal of the catheter.

Also disclosed as the catheter tube having an internal-layer tube wound with a strand as the reinforcing-material layer in Patent Document 2 is a catheter tube having a long tube-shaped part consisting of a proximal end, a distal end, and a hollow passage extending between these ends, wherein: the long tube-shaped part has an external tube-shaped cover of a first cover material; an internal tube-shaped liner of a first liner material coaxial therewith; and at least one layer of a first ribbon-reinforcing material wound around the internal tube-shaped liner spirally or coaxially and covered with the external tube-shaped cover.

However, the degree of latitude in controlling the rigidity/flexibility inclination is lower even in the configuration, and the elastic force of the ribbon-reinforcing material often leads to breakage of the internal tube-shaped liner or the external tube-shaped cover by the cutoff edge during production, lowering productivity. In addition, the rigidity/flexibility balance of the catheter tube is not intended to be adjustable according to access route. Further, as for the marker for X-ray radiopaque, a resin containing a blended X-ray impermeable powder is placed on the distal end of the catheter, but it is not possible to assure the favorable X-ray radiopaque and high flexibility in the distal region at the same time in the configuration. In addition, no attention is give to the elongation during repeated insertion and withdrawal of the catheter.

Yet alternatively, Patent Document 3 discloses a catheter comprising a flexible tube-shaped catheter main body and a reinforcing coil embedded in the wall of the catheter main body, wherein: the catheter main body further has a first region at the distal end of the catheter and a second region at a position closer to the proximal end from the first region; the coil extends from the first region to the second region; the coil is wound at a relatively large winding pitch over the entire length in the second region; the coil is wound at a relatively smaller winding pitch between neighboring coils over the entire length in the first region; the coil winding pitch declines gradually in the direction toward the distal end; and the rigidity of the catheter is smaller in the first region than in the second region.

However, although it is possible to form a high-rigidity proximal region and a high-flexibility distal region in the catheter tube and keep a favorable balance in flexural rigidity, the rigidity/flexibility balance of the catheter tube is not intended to be adjustable according to access route. Further in the catheter tube, the entire reinforcing coil is an X-ray impermeable metal wire; the flexibility of the distal region is insufficient; and the X-ray radiopaque is excessively high, causing troubles in decision making by surgeons during operation. In addition, no attention is give to the elongation during repeated insertion and withdrawal of the catheter.

In addition, Patent Document 4 discloses, as a catheter tube a having reinforcing-material layer as braided around an internal layer tube, a vascular catheter, comprising a proximal region, a distal region, and a long shaft having a lumen extending between them, wherein: the proximal region has an internal smooth polymer layer, a reinforcement layer and external layer; respective layers have distal ends; the reinforcement layer is a braid of a metal part having multiple polymer members; and each polymer member contains multiple monofilaments.

However, although it is possible to form a rigid proximal region and a flexible distal region on the catheter tube, the degree of latitude in controlling the rigidity/flexible inclination is low. In addition, the X-ray visible marker used is a marker formed by covering the internal layer tube with a metal sheet or tube, and thus, it is not possible to ensure high flexibility of the catheter distal regions in the area in contact with the marker or the area close to it. Further, the catheter tube, although made resistant to elongation, is larger in diameter, because the catheter is wound with braid, which may make it difficult to insert it into the narrow blood vessel.

Alternatively for preparing a catheter in which a braid of strand is wound around an internal-layer tube as the reinforcing material layer, Patent Document 5 discloses a method of producing a catheter comprising: forming a torque-transmitting region by covering the external surface of a thermoplastic tube containing an inserted metal core wire with a metal braid; forming multiple insertion distal regions having a constant width at a particular gap in the tube machine direction by removing part of the braid intermittently in the machine direction by irradiation of a laser beam at a wavelength of 1.06 µm from outside; withdrawing the metal core wire; and forming the insertion distal regions continuously in the distal regions of torque transmission regions by dividing the tube into pieces at the terminal of each insertion distal region.

However, the step of removing a part of the braid intermittently in the machine direction by irradiation of a laser beam at a wavelength of 1.06 µm is very complicated. The method is also lower in productivity, because, when a torque transmission region is formed continuously by applying a metal braid continuously over the external surface of a thermoplastic tube having an inserted metal core wire and embedding the braid into the outer surface of the tube by softening by heating to a depth of about 1/2 to 1/5 of the thickness in the downstream step, the metal braid may cause problems such as ejection of the metal braid out of the tube surface caused by the bending of cut edge due to its elastic force during embedment of the braid by heat softening of the tube. It is also difficult to control the rigidity/flexibility inclination sufficiently. In addition, the rigidity/flexibility balance of the catheter tube is not intended to be adjustable according to access route. In addition, no attention is given to the elongation during repeated insertion and withdrawal of the catheter.

Alternatively, Patent Document 6 discloses a catheter, having a manifold, a proximal end shaft unit connected to the manifold, a distal end shaft unit connected to the terminal of the proximal end shaft unit that is higher in flexibility compared to the proximal end shaft unit, and a braid unit formed around the distal end shaft unit, wherein the braid unit, which is formed on the distal end shaft unit, contains numerous filaments, forming approximately 70 to 120 picks per inch, each containing multiple filaments, by mutual crossing.

However, the rigidity inclination of the catheter tube is not considered, in relation with the change of the pick interval and also with the hardness of the external layer resin, and thus, the rigidity/flexibility inclination is not controlled sufficiently. In addition, there is no particular attention given to the thickness of the catheter, and the rigidity/flexibility balance of catheter tube is not adjustable according to the access route. Further, no attention is give to the elongation during repeated insertion and withdrawal of the catheter.
Patent Document 1: Japanese Patent No. 3,310,031
Patent Document 2: Japanese Patent No. 2,672,714
Patent Document 3: Japanese Unexamined Patent Publication No. 2001-218,851
Patent Document 4: Japanese Unexamined Patent Publication No. 2002-535,049
Patent Document 5: Japanese Unexamined Patent Publication No. 2000-225,194
Patent Document 6: Published Japanese Translation of a PCT Application No. 11-506,369

### SUMMARY OF THE INVENTION

### Problems to be Solved by the Invention

An object of the present invention is to provide a medical catheter tube superior in positioning efficiency, torque-transmitting efficiency, flexibility, kink resistance, pressure resistance, insertion efficiency, X-ray radiopaque, and others, and a production method thereof. Another object of the present invention is to provide a thin-layered medical catheter tube that is resistant to deterioration in the accuracy of catheter position caused by elongation when the medical catheter tube is inserted and withdrawn repeatedly by a surgeon and allows insertion thereof into narrow blood vessel, and a production method thereof.

### Means to Solve the Problems

The present invention relates to a medical catheter tube, comprising
an internal-layer tube of resin,
first and second reinforcing-material layers respectively of first and second wires formed on the internal-layer tube,
a marker of an X-ray impermeable metal formed covering the internal-layer tube, and
an external-layer tube of resin covering the internal-layer tube, the reinforcing-material layer, and the marker, wherein:
   the catheter tube has a proximal region, a distal region, and a most distal region from the proximal terminal;
   the first and second wires are made of a synthetic resin wire and/or a metal wire;
   the first wire forms the first reinforcing-material layer formed in the catheter axial line direction;
   the second wire forms the second reinforcing-material layer covering the first reinforcing-material layer as wound in the coil shape in the catheter circumferential direction;
   the marker is formed in the distal region;
   the marker is flexible to bending deformation; and
   the flexural rigidity of the reinforcing-material layer and the external-layer tube decreases stepwise or continuously in the direction from the proximal to distal region.

Preferably in the present invention, the resin tube of the internal-layer tube is lubricant and flexible; the first and second wires provides the internal-layer tube with kink resistance, pressure resistance, torque-transmitting efficiency, and insertion efficiency; the internal-layer tube, the reinforcing-material layer, the marker, and the external-layer tube are integrated; the distal region is formed in the region distal from the reinforcing-material layer; and the most distal region does not have the reinforcing-material layer or the marker.

The present invention also relates to the medical catheter tube above, wherein the marker is a coil of an X-ray impermeable metal wire wound in the coil shape around the internal-layer tube, a tube of an X-ray impermeable metal sheet having a slit and covering the internal-layer tube, or a tube formed by using a resin containing a blended X-ray impermeable metal powder. In the catheter above, the X-ray impermeable metal sheet is preferably a square X-ray impermeable metal sheet having slits from both sides.

The present invention relates to the medical catheter tube above, wherein the first and second wires forming the reinforcing-material layer are a synthetic fiber having a thermoplastic liquid crystal polymer as its internal core and a flexible polymer as its sheath.

The present invention relates to the medical catheter tube above, wherein the winding pitch of the second wire of a synthetic resin wire and/or a metal wire forming the second reinforcing-material layer is changing continuously or stepwise in the direction from the proximal to distal region.

The present invention relates to the medical catheter tube above, wherein the external-layer tube has multiple segments; and the multiple segments are so aligned that the Shore D hardness of the multiple segments decreases stepwise in the direction from the proximal to distal region.

The present invention relates to the medical catheter tube above, wherein the internal- and external-layer tubes are bound to each other via the reinforcing-material layer and the marker.

The present invention further relates to the medical catheter tube above, wherein the internal-layer tube is made of a resin lubricant to the guide wire and others extending therein.

The present invention relates to the medical catheter tube above, wherein the external-layer tube in the most distal region is molded into a rounded or tapered shape in which the external diameter thereof changes.

The present invention also relates to the medical catheter tube above, wherein the external-layer tube is coated to be hydrophilic.

The present invention further relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the reinforcing-material layers are formed by placing a synthetic resin wire and/or a metal wire in the catheter axial line direction and winding a synthetic resin wire and/or a metal wire in the coil shape continuously, stepwise or at varying pitches in the catheter circumferential direction.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the external-layer tube is so formed on the internal-layer tube having the reinforcing-material layers and the marker that the Shore D hardness of the resin tube thereof decreases stepwise in the direction from the proximal to distal region; the internal-and external-layer tubes are connected to each other via the reinforcing-material layer and the marker by heat shrinkage of the shrink tube on the external surface; and the shrink tube is removed after cooling.

The present invention also relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, forming multiple resin regions different in Shore D hardness in the external-layer tube, and adjusting the length of the resin regions different in Shore D hardness; the internal- and external-layer tubes are connected to each other via the reinforcing-material layer and the marker by heat shrinkage of the shrink tube; and the shrink tube is removed after cooling.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the external-layer tube is so extruded by switching extrusion method that the Shore D hardness decreases stepwise or continuously in the direction from the proximal to distal region on the internal-layer tube having the reinforcing-material layers and the marker formed; the internal- and external-layer tubes are connected to each other via the reinforcing-material layer and the marker by heat shrinkage of the shrink tube on the external surface; and the shrink tube is removed after cooling.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, forming multiple resin regions different in Shore D hardness in the external-layer tube by switching extrusion method, and adjusting the length of the resin regions different in Shore D hardness; the internal- and external-layer tubes are connected to each other via the reinforcing-material layer and the marker by heat shrinkage of the shrink tube; and the shrink tube is removed after cooling.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the external layer is extruded into multiple regions different in Shore D hardness formed on the internal-layer tube having the reinforcing-material layers and the marker by coat-switching extrusion molding in such a manner that the Shore D hardness decreases stepwise or continuously in the direction from the proximal to distal region; and the internal-layer tube, the reinforcing-material layer, the X-ray impermeable marker, and the external-layer tube are integrated.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, extruding the external layer on the internal-layer tube having the reinforcing-material layers and the marker by coat-switching extrusion molding while changing the Shore D hardness thereof stepwise, adjusting the length of the resin regions different in Shore D hardness; and the internal-layer tube, the reinforcing-material layer, the X-ray impermeable marker, and the external-layer tube are integrated

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the X-ray impermeable marker is formed by winding an X-ray impermeable metal wire in the coil shape on the internal-layer tube in the region close to the distal side of the reinforcing-material layer, covering it with a square X-ray impermeable metal sheet having slits from both sides, or using a resin containing a blended X-ray impermeable metal powder, and the catheter is flexible in the distal region.

The present invention relates to a method of producing the medical catheter tube according to the present invention, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the most distal region of the external-layer tube is molded into a rounded or tapered shape.

### Advantageous Effects of the Invention

The present invention provides, by the means to solve the problems above, a medical catheter tube superior in many properties such as movement in accordance with guide wire, positioning efficiency, continuous change in flexibility in the direction from the proximal to distal region, degree of latitude in adjusting rigidity/flexibility balance, determination of the rigidity/flexibility balance according to access route, kink resistance to complicated bent without crimping, pressure resistance, movement in accordance with guide wire, and productivity.

Particularly advantageously, the present invention provides a thin-layered medical catheter tube favorable in X-ray radiopaque and also in flexibility that is resistant to deterioration in the accuracy of catheter position caused by elongation when the medical catheter tube is inserted and withdrawn repeatedly by a surgeon and allows insertion thereof into the narrow blood vessel, and a production method thereof.

### BRIEF DESCRIPTION OF THE DRAWINGS

Figure 1 is a flowchart showing the production procedure.
Figure 2 is a schematic view illustrating a metal core wire wound around reels.
Figure 3 is a schematic view illustrating an internal-layer tube being formed continuously by an extruder.
Figure 4 is a schematic view illustrating a reinforcing-material layer being formed by placing a reinforcing material wire on the internal-layer tube in the axial direction and additionally winding the tube with another reinforcing material wire.
Figure 5 is an expanded side view illustrating the catheter, showing the pitch of the reinforcing material wire wound in the coil shape.
Figure 6 is a crosssectional view illustrating a wire favorably used as a synthetic resin wire; Figure 6(A), an expanded perspective view of the wire terminal; and Figure 6(B), a scanning micrograph of the wire terminal.
Figure 7 is a schematic view illustrating the state where the internal-layer tube and the reinforcement layer are removed in the region corresponding to the catheter distal and proximal regions.
Figure 8 is a schematic side view illustrating an individual catheter.
Figure 9 is a partial schematic side view illustrating the state where a X-ray impermeable metal wire marker is placed on the catheter distal end.
Figure 10 is a side view illustrating a square X-ray impermeable metal sheet marker having slits from both sides.
Figure 11 is a partial schematic side view illustrating a square X-ray impermeable metal sheet marker having slits from both sides, placed on the catheter distal end.
Figure 12 is a partial schematic side view illustrating a resin tube containing a blended X-ray impermeable metal powder placed on the catheter distal end.
Figure 13 is an expanded side view illustrating the state where the internal-layer tube and the reinforcement layer are removed in the region corresponding to the catheter distal and proximal regions and an X-ray impermeable metal wire marker is placed thereon.
Figure 14 is an expanded side view illustrating the state where the internal-layer tube and the reinforcement layer are removed in the region corresponding to the catheter distal and proximal regions and an X-ray impermeable metal sheet marker is placed thereon.
Figure 15 is an expanded side view illustrating the state where the internal-layer tube and the reinforcement layer are removed in the region corresponding to the catheter distal and proximal regions and a resin tube containing a blended X-ray impermeable metal powder is placed thereon.
Figure 16 is a schematic crosssectional side view illustrating the state where four kinds of resin tubes different in Shore D hardness for the external layer are placed densely to each other.
Figure 17 is a schematic crosssectional side view illustrating an external-layer tube wherein the Shore D hardness changes stepwise.
Figure 18 is a schematic sectional view illustrating the state where a shrink tube is formed.
Figure 19 is a schematic sectional view illustrating a catheter pulled through a circular hole of a heated mold.
Figure 20 is a schematic sectional view illustrating the state where the internal-layer tube, the reinforcing-material layer, and the external-layer tube are integrated by shrinkage of the shrink tube and the terminal of the external-layer resin tube is molded into a rounded shape.
Figure 21 is a schematic sectional view illustrating the terminal of a tube and a heated mold for molding the terminal.
Figure 22 is a schematic sectional view illustrating the state where the terminal of the tube is heat-molded in contact with the mold.
Figure 23 is a schematic sectional view illustrating the state where the shrink tube is removed.
Figure 24 is a schematic view illustrating a continuous catheter prepared by welding metal core wires that is wound around a reel.
Figure 25 is a schematic view illustrating the state where an external layer is being formed by coating extrusion.
Figure 26 is a schematic sectional view illustrating an individual catheter, after cutoff, retaining a shrink tube at the distal end.
Figure 27 is a schematic sectional view illustrating the state where the metal core wire is withdrawn and the proximal terminal is finished.
Figure 28 is a conceptual diagram showing the rigidity/flexibility balance.

### EXPLANATION OF REFERENCES

- 1: Metal core wire
- 2: Reel
- 3: Internal-layer tube
- 4: Extruder
- 5: Reinforcing-material layer
- 51: First reinforcing-material layer
- 52: Second reinforcing-material layer
- 6: Bobbin for placing the first wire on catheter in the axial direction
- 61: First wire
- 7: Bobbin for winding the second wire in the catheter circumferential direction
- 71: Second wire
- 8: Revolving unit
- 9: Core of thermoplastic liquid crystal polymer
- 10: Island (sheath) of thermoplastic liquid crystal polymer
- 11: Sea (sheath) of flexible polymer
- 12: Metal core wire
- 13: X-ray impermeable metal wire marker
- 14: Square X-ray impermeable metal sheet marker having slits from both sides
- 15: Square X-ray impermeable metal sheet marker having slits from both sides that are attached
- 16: Resin tube containing a blended X-ray impermeable metal powder
- 17: Resin tube containing a blended X-ray impermeable metal powder without slit
- 18: Metal core wire
- 19: X-ray impermeable metal wire marker
- 20: Square X-ray impermeable metal sheet marker having slits from both sides that are attached
- 21: Resin tube containing a blended X-ray impermeable metal powder
- 22: External-layer tube
- 22a: Maximum-Shore D hardness external-layer tube
- 22b: High-Shore D hardness external-layer tube
- 22c: Low-Shore D hardness external-layer tube
- 22d: Minimum-Shore D hardness external-layer tube
- 23: X-ray impermeable marker
- 24: Shrink tube
- 25: Heated mold having an open circular hole
- 26: Rounded region
- 27: Heated mold
- 28: Heat-molded tapered region
- 29: Spot welding machine
- 30: Extrusion die
- 31: Extruder
- 32: Shrink tube

### BEST MODE OF CARRYING OUT THE INVENTION

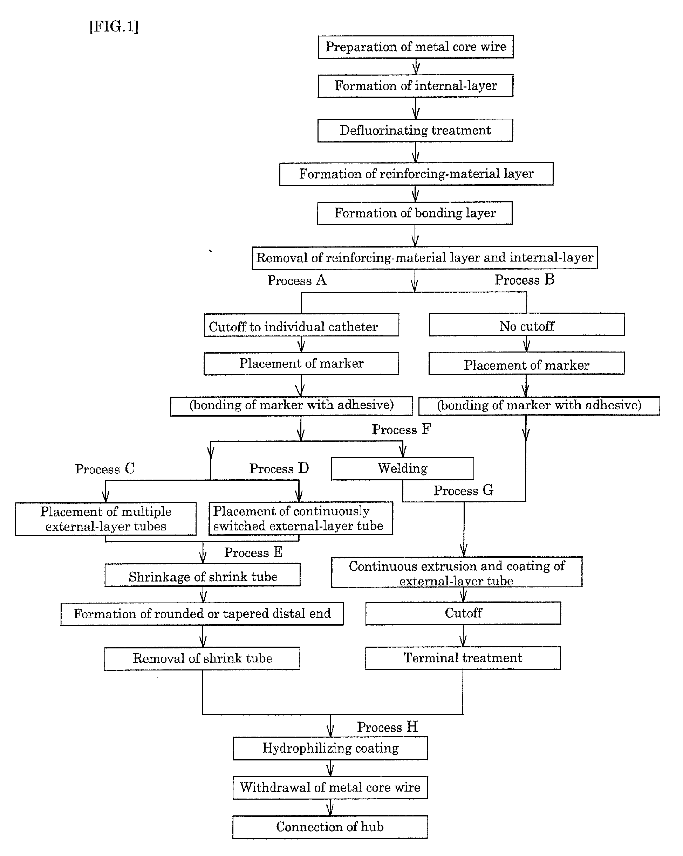

Hereinafter, the best mode and configuration of a medical catheter tube and a method of producing the same according to the present invention will be described with reference to drawings. These drawings are schematic drawings showing the characteristics of the configuration of the present invention, and the length and diameter of each region is arbitrary, if the catheter tube can be used favorably as a medical catheter tube. Figure 1 is a flowchart showing a production procedure, and the mode and configuration of the catheter and also the production method thereof according to the present invention will be described with reference to the Figure. Various modifications of the mode and configuration of the catheter and the production method thereof according to the present invention are possible within the scope of the present invention described in Claims.

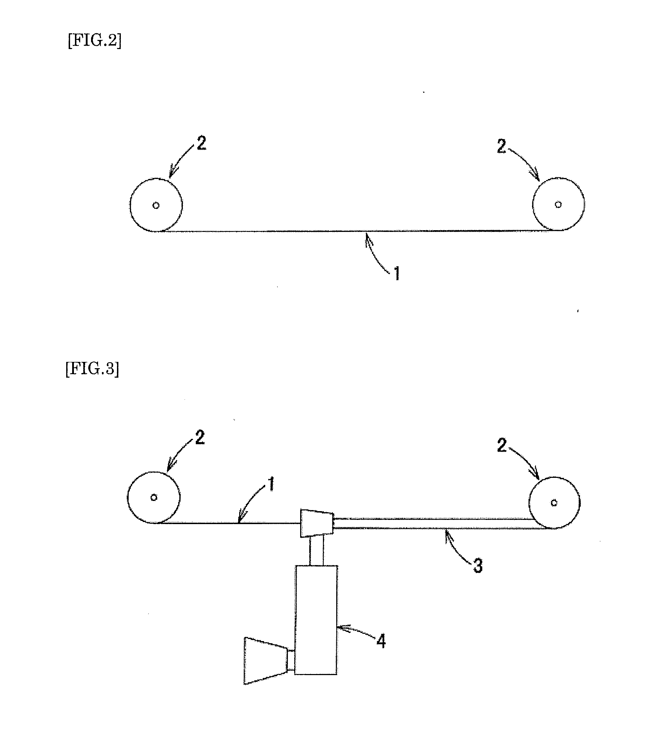

A metal core wire 1 is first made available, as shown in Figure 2. The metal core wire 1 is wound around reels 2; the external diameter of the wire corresponds roughly to the internal diameter of the catheter to be produced; and it is preferably a metal-plated copper wire or a stainless steel wire. For convenience, in all Figures 2 and below, the left side of the wire represents the proximal region, while the right side, the distal region.

As shown in Figure 3 an internal layer tube 3 is formed on the metal core wire 1 with an extruder 4 by extrusion coating.

Examples of the materials for the internal-layer tube 3 include fluorine resins such as polytetrafluoroethylene, tetrafluoroethylene-perfluoroalkyl vinylether copolymers, tetrafluoraethylene-hexafluoropropylene copolymers, and ethylene-tetrafluoroethylene copolymers; polyolefins such as polypropylene, polyethylene, and ethylene-vinyl acetate copolymers; polyamides; polyesters such as polyethylene terephthalate and polybutylene terephthalate; polyurethane, polyvinyl chloride, polystyrene resins, polyimide, and other resins, and the mixture thereof, but fluorine resins such as polytetrafluoroethylene and tetrafluoroethylene-perfluoroalkyl vinylether copolymers are preferable for making the final product more lubricant, for example, to the guide wire extending in the internal layer tube 3 and favorable in positioning efficiency and guide wire compatibility. When polytetrafluoroethylene is used, the additives therein are processed, for example, by drying, and the resin is burned.

The internal layer tube 3 that coats the metal core wire 1 preferably has sufficiently high adhesiveness to the metal core wire 1. For the purpose of increasing the adhesiveness between the internal layer tube 3 and an external-layer tube 22 formed later, the surface of the internal layer tube may be roughened or modified mechanically (for example, abrasion of internal layer tube surface with a sand paper) and/or chemically (use of a defluorinating agent such as sodium naphthalene in dimethylether), and/or electrically, for example by plasma processing, in the subsequent step of coating the external-layer tube 22.

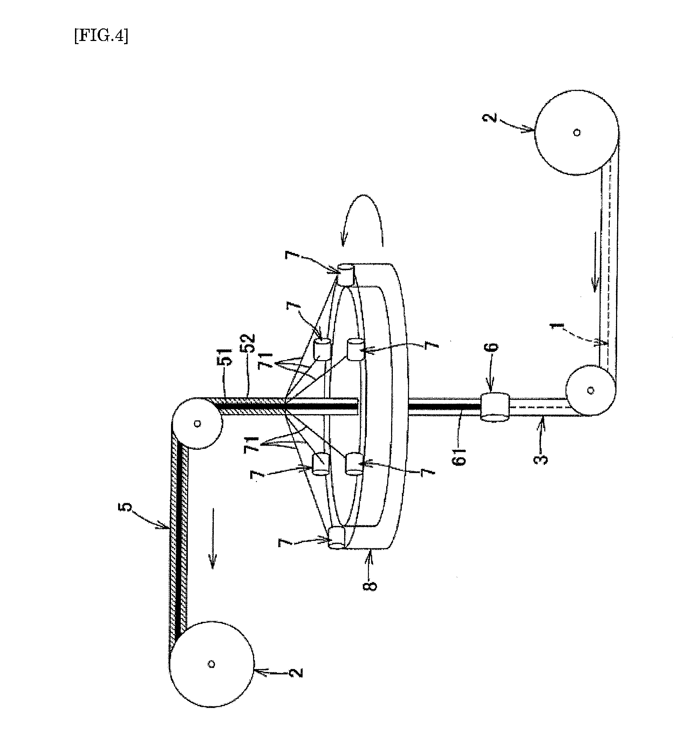

Then, a reinforcing-material layer 5 is formed in a device similar to that shown in Figure 4. In the device, the metal core wire 1 covered with the internal-layer tube 3 formed in Figure 3 is sent upward vertically; a first wire 61 supplied from a bobbin 6 is placed on the catheter in the shaft line direction; and second wires 71 supplied from multiple bobbins 7 on a revolving unit 8 by revolution are wound around the catheter in the catheter circumferential direction in a coil shape, while covering the first wire 61 placed in the shaft line direction. A first reinforcing-material layer 51 of the first wire 61 extending in the catheter axial direction plays roles of improving the insertion efficiency of catheter and preventing deterioration in the accuracy of catheter position by catheter elongation when it is pulled. Alternatively, a second reinforcing-material layer 52 of coil-shaped second wires 71 extending in the catheter ciucumferential direction plays roles, for example, of giving pressure resistance and controlling the flexibility of catheter.

One or more wires may be wound around each bobbin 6 or 7 and used for forming the catheter reinforcing-material layer 5. The first reinforcing-material layer 51 is formed with only one first wire 61 or only one bundle thereof in the catheter axial direction by using only one bobbin 6 in the Figure, but instead, multiple wires or multiple bundles may be placed in the axial direction by using multiple bobbins 6. In addition, second wires 71 from six bobbins 7 are wound in the coil shape, forming the second reinforcing-material layer 52 in Figure 4, but the number of the wires or the bobbins may be arbitrary. Multiple wires wound around the bobbin 6 or 7 are preferably placed or wound around catheter in the flat state without mutual crossing, for prevention of increase of the external diameter.

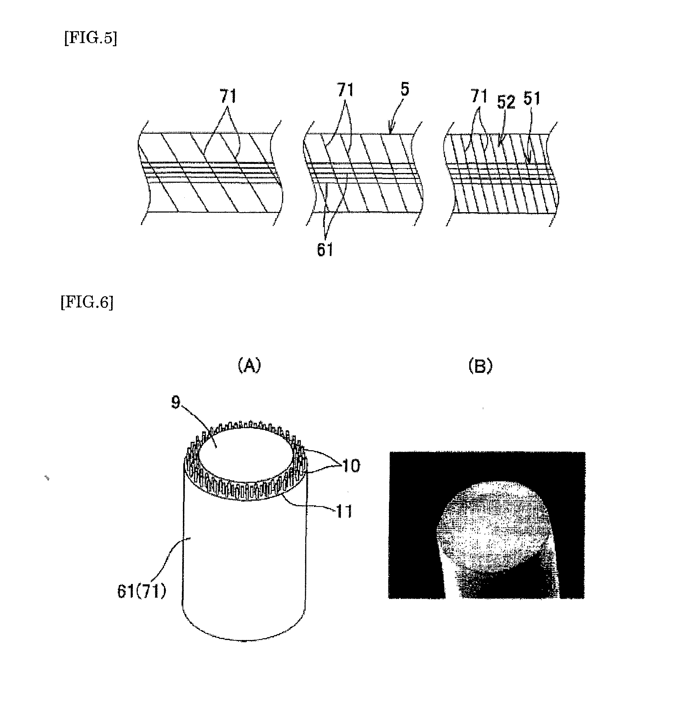

The second wires 71 are wound in the coil state in the catheter circumferential direction at a constant interval while the metal core wire 1 is fed and rotated at constant speeds, or the revolving unit 8 is rotated at a constant rotational velocity in the catheter circumferential direction. As shown in the expanded view of Figure 5 showing winding patterns around one catheter, it is possible to wind the wire densely in the catheter distal region and coarsely in the proximal region, by changing the feed speed of metal core wire and/or the rotational velocity of revolving unit 8. Dense winding leads to higher flexibility, while coarse winding to higher rigidity.

A metal wire may be used together with a synthetic resin wire as the first wire 61 or the second wire 71. Particularly favorably used as the synthetic resin wire is a wire having a core 9 of a thermoplastic liquid crystal polymer and a coating layer containing islands of a thermoplastic liquid crystal polymer (sheath) 10 and a sea of a flexible polymer (sheath) 11, as shown in the schematic crosssectional view of Figure 6(A) and the scanning micrographs shown in Figure 6(B). In an example of the synthetic resin wire, the thermoplastic liquid crystal polymer is polyarylate, and the flexible polymer is polyethylene naphthalate. The diameter of the synthetic resin wire favorably used is preferably 5 to 50 µm. Examples of the wires 61 and 71 are disclosed in Japanese Unexamined Patent Publication No. 2002-20932.

Examples of the other synthetic resins for the wire for use in the present invention include polyesters such as polyethylene terephthalate, polybutylene terephthalate, and polymethylene terephthalate; polyolefins such as polyethylene and polypropylene, hard polyvinyl chloride, polyamide, polyimide, polystyrene, thermoplastic polyurethane, polycarbonate, ABS resins, acrylic resins, polymethyl methacrylate, polyacetal, polyarylates, polyoxymethylene, high-tension polyvinylalcohol, fluoroplastics, polyvinylidene fluoride, polytetrafluoroethylene, ethylene-vinyl acetate hydrolysates, polysulfone, polyether sulfone, polyether ketone, polyphenyleneoxide, polyphenylene sulfide, aromatic polyaramides such as Kevlar (registered trade name of E.I. du Pont de Nemours and Company in U.S.). Polymer alloys containing at least one of the resins above, carbon fiber, and glass fiber and the like.

Examples of the metal wire include various metal wires such as of stainless steel, copper, tungsten, nickel, titanium, music wire, Co-Cr alloy, Ni-Ti alloy, Ni-Ti-Co alloy, Ni-Al alloy, Cu-Zn alloy, or superelastic alloy such as Cu-Zn-X alloy (for example, X is Be, Si, Sn, Al, or Ga), and amorphous alloys; and, among these materials, use of stainless steel wire is preferable for making the radiopaque thereof lower than that of X-ray-impermeable marker for ensuring radiopaque of the X-ray-impermeable marker later placed and also for assuring better processability, cost, and safety. The metal wire preferably has a diameter of approximately 5 to 50 µm.

The synthetic resin wire and the metal wire may be used as a single wire or in combination (e.g., twisted or bundled wire).
In the present invention, only a synthetic resin wire or a metal wire may be used, or both synthetic resin and metal wires are used in combination.

After the reinforcing-material layer 5 is formed, a bonding layer not shown in the Figure may be formed additionally to fix it to the internal layer tube 3. The bonding layer, which is aimed at blocking small pores generated in the internal layer tube 3 and improving anti-bursting strength, is formed by coating or spray-coating a soft polyurethane, a polyurethane dispersion or a soft adhesive agent on the internal layer tube 3 and the braided reinforcing-material layer 5 to a thickness of 5 to 50 µm.

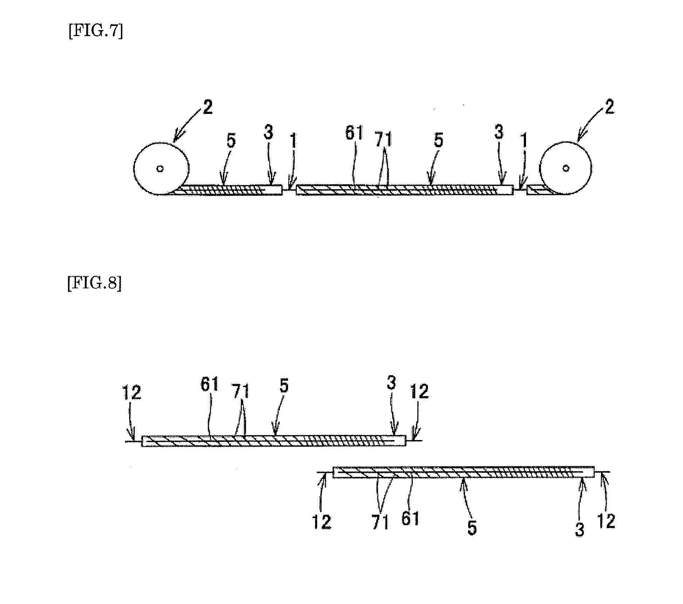

Subsequently as shown in Figure 7, the internal layer tube 3 and the reinforcing-material layer 5 at the positions corresponding to the distal and proximal regions of the catheter are removed, leaving the metal core wire 1 exposed.

Subsequently as show in the flowchart of Figure 1, a process A or B may be carried out. The process A will be described first.

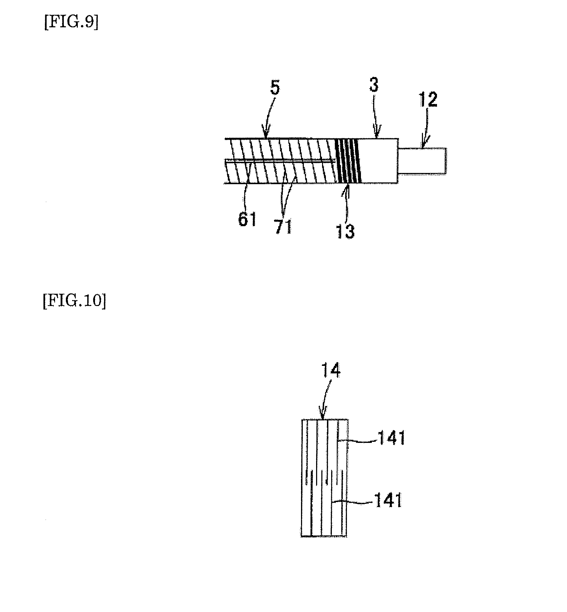

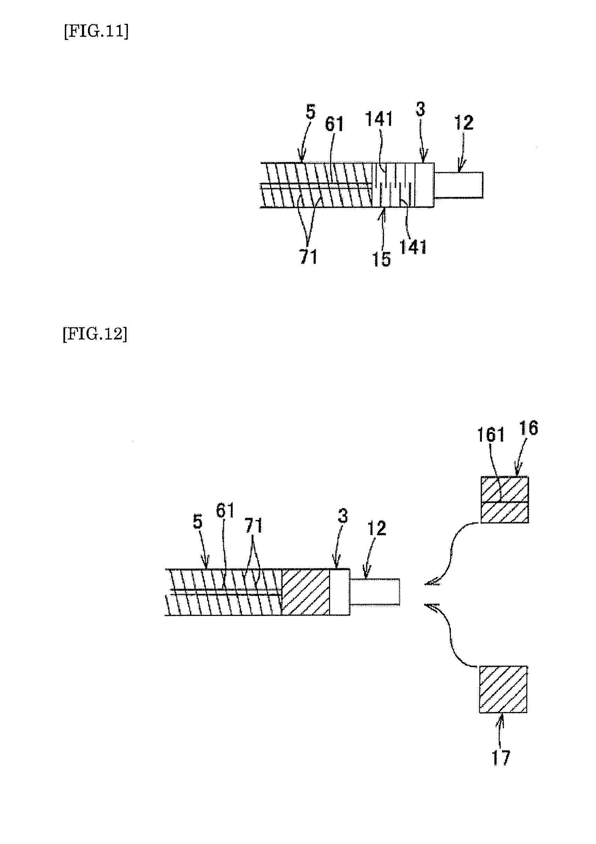

In the process A, a catheter is formed by cutting the exposed metal core wire 1, as shown in Figure 8. The catheter tube cut has at least a proximal region, a distal region, and a most distal region from the proximal end. Figure 9 is an expanded view of the catheter distal region, while Figure 12 is a view illustrating the metal core wire after cutoff. An X-ray-impermeable metal wire marker 13 is placed densely at the distal end of the reinforcing-material layer 5, and, for that purpose, an X-ray-impermeable metal wire is wound as a coil around the internal layer tube 3 in the distal region of the catheter tube. The X-ray-impermeable metal wire may be wound around the internal layer tube 3 densely with the metal wires in contact with each other or coarsely with the metal wires separated from each other. In Figure 9, it is wound in the same direction with the second wire 71 of reinforcing-material layer 5, but may of course be wound in the opposite direction. An X-ray-impermeable metal sheet marker 14 with slits 141 from both sides in the shape shown in Figure 10 is placed as it wraps the internal layer tube 3 to the distal side of the reinforcing-material layer 5 in the catheter distal region, as shown in Figure 11. Figure 11 is an expanded view of the catheter distal region, in which the numeral 15 represents an X-ray-impermeable metal sheet marker 14 that wraps the internal layer tube 3.

The diameter of the X-ray-imparmeable marker is preferably 5 to 50 µm when a metal wire is used, and the thickness thereof is preferably 5 to 30 µm when a metal sheet is used. The X-ray-impermeable marker shows favorable flexibility, when a metal wire or a metal sheet is used. A metal higher in X-ray impermeability and X-ray radiopaque such as platinum (Pt), a Pt-Ir alloy, a Pt-W alloy, a Pt-Ni alloy, gold, or silver is used favorably as the material for the X-ray-impermeable marker.

As shown in Figure 12, a resin tube containing a blended X-ray-impermeable metal powder such as barium sulfate, bismuth oxide, bismuth subcarbonate, bismuth tungstate, or bismuth-oxychloride may be formed in the region to the distal side of the reinforcing-material layer 5 on the internal layer tube 3. The resin for use is preferably the same as that for the external-layer tube described below. The resin tube 16 containing a blended X-ray-impermeable metal powder may be placed then, as it is slits 161 in the axial direction as shown in Figure 12, or the resin tube 17 may be placed in the original tube shape, as shown in Figure 12. The thickness of the resin tube 16 or 17 containing the X-ray impermeable metal powder is preferably 5 to 30 µm. As will be described later, the distal region of the external-layer tube 22 may be formed with a resin containing an X-ray impermeable metal powder. Alternatively, the X-ray impermeable marker 13, 15, 16, or 17 may be fixed onto the internal-layer tube 3 as needed, for example, by using an adhesive agent.
These are processings performed in the process A.

The process B is a process of placing an X-ray-impermeable marker without cutting of the catheter.

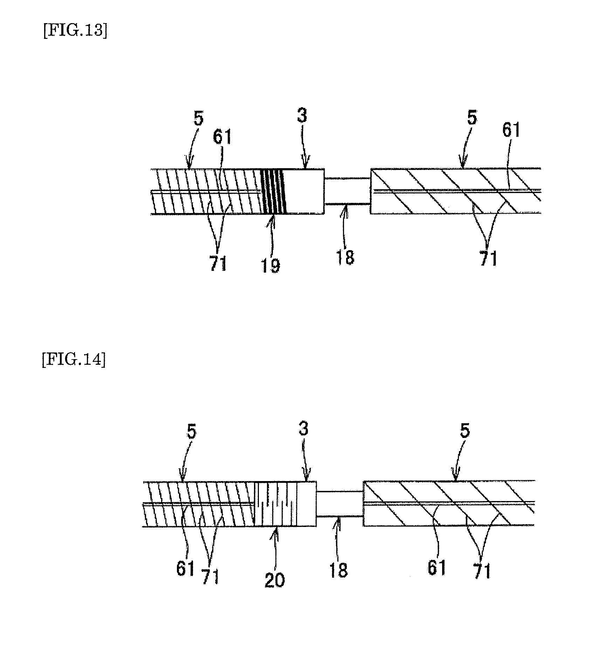

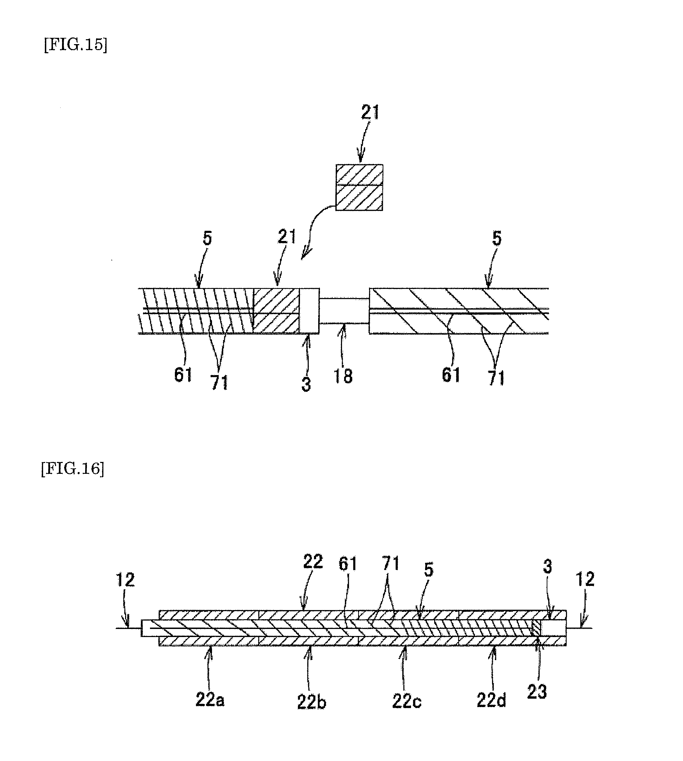

Figure 13 is an expanded view illustrating the catheter proximal region shown in Figure 7. 18 represents a metal core wire, and an X-ray impermeable metal wire marker 19 is wound around the internal-layer tube 3 densely in the region distal from the reinforcing-material layer 5. The X-ray-impermeable metal wire may be wound around the internal layer tube densely with the metal wires in contact with each other or coarsely with the metal wires separated from each other. Alternatively, an X-ray-impermeable metal sheet marker 20 having slits from both sides in the shape shown in Figure 10 is placed as it wraps the internal layer tube 3 in the region distal from the reinforcing-material layer 5, as shown in Figure 14. The shape and the material for the X-ray-impermeable marker are the same as those described above. In addition, a resin tube containing a blended X-ray impermeable metal powder 21 having a slit in the axial direction may be placed on the internal-layer tube 3 distal from the reinforcing-material layer 5, as shown in Figure 15.
These are processings performed in the process B.

The process C is a step of forming an external-layer tube 22 on the catheter prepared in process A. The flexural rigidity of the external-layer tube 22 should decrease stepwise or continuously in the direction from the proximal region to the distal region. The degree of the flexural rigidity in the present description corresponds to the value of Shore D hardness of the resin for the external-layer tube 22.

The external-layer tube 22 is formed in such a manner that resin tubes 22a to 22d for the external-layer tube 22 have Shore D hardnesses changing stepwise from the proximal region to the distal region, as shown in Figure 16. In the distal region, the resin tube is formed beyond the X-ray-impermeable marker 23. Preferably, the external layer resin tube 22 has multiple segments, and the multiple segments are aligned in such a manner that the Shore D hardnesses of the resins for the segments decrease stepwise in the direction from the proximal region to the distal region. The Shore D hardness in the present description is a value determined with a type-D durometer according to ISO 7619. Four kinds of segments different in Shore D hardness are placed densely in contact with each other in Figure 16 in such a manner that the Shore D hardness decreases gradually in the direction from the proximal region to the distal region.

Thus, the Shore D hardness of the resin tubes for the external-layer tube 22 is in the order of 22a > 22b > 22c > 22d in Figure 16. Resins having a Shore D hardness of approximately 20 to 80 are used favorably. When an external-layer tube 22 made of a single resin having a particular Shore D hardness is placed, the external-layer tube 22 having the single kind of Shore D hardness may be cut into pieces and then placed thereon densely. Preferably, there is a very thin gap between the composite of the internal layer tube 3 and the reinforcing-material layer 5 braided thereon and the resin tube of external-layer tube 22, and in such a configuration, the wire for the reinforcing-material layer 5 is less disturbed. The resin tubes for the external-layer tube 22 different in Shore D hardness allows gradual change of the catheter tube in the rigidity/flexible inclination when placed at positions separated from the position where the pitch distance of braid is changing. When there are multiple regions different in Shore D hardness, it is possible to control the rigidity/flexibility balance in a wider range by adjusting the length of the each region different in Shore D hardness and also the winding pitch of the wire.

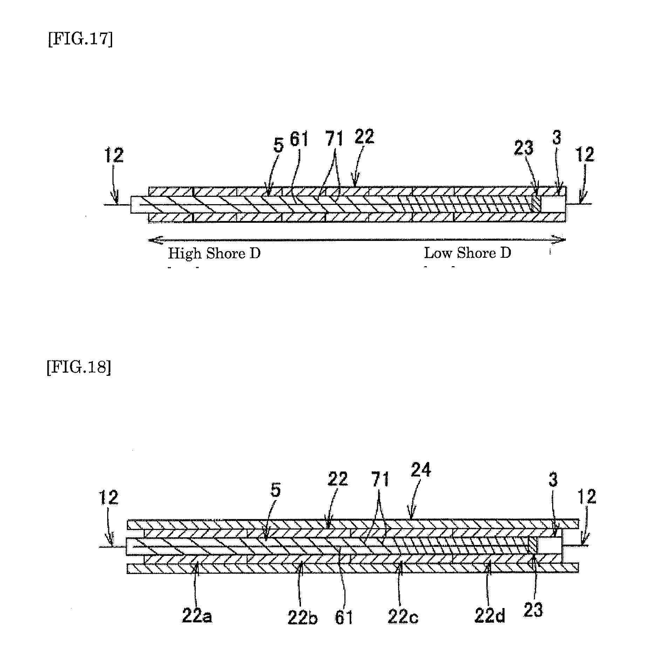

In another process D, the resin tube for the external-layer tube 22 may be prepared and placed by preparing a resin tube having a Shore D hardness changing stepwise by using plural extruders connected to an extrusion die and feeding the resins different in Shore D hardness from the plural extruders one by one by operation and termination, and placing the resin tube around the internal layer tube 3 having a braided reinforcing-material layer 5, as shown in Figure 17. Yet alternatively, multiple resin tubes having Shore D hardnesses changing stepwise may be prepared by connecting plural extruders to a mold having a valve mechanism and continuously feeding resins different in Shore D hardness into the extrusion channel while extrusion and discharge are switched, and these resin tubes, placed around the internal layer tube 3 having a braided reinforcing-material layer 5, as shown in Figure 17. Then, the external-layer tubes 22 should be so placed that the external layer has a higher Shore D hardness in the region closer to the proximal end and a lower Shore D hardness in the region closer to the distal end. When there are multiple regions different in Shore D hardness, it is possible to control the rigidity/flexibility balance in a wider range by changing the winding pitch of the wire and adjusting the length of the regions different in Shore D hardness. Although not shown in the Figure, the most distal region of the resin tube may be formed with a resin containing a blended X-ray-impermeable metal powder in these methods.

Examples of the materials for the resin tubes of the external-layer tube 22 include various elastomers such as polyamide elastomer, polyester elastomer, polyurethane-elastomer, polystyrene elastomer, fluorine-based elastomer, silicone rubber, and latex rubber, and two or more of them may be used in combination.

The polyamide elastomer is a concept including block copolymers having a hard segment of an aliphatic or aromatic polyamide such as of nylon 6, nylon 64, nylon 66, nylon 610, nylon 612, nylon 46, nylon 9, nylon 11, nylon 12, an N-alkoxymethyl-modified nylon, a hexamethylenediamine-isophthalic acid condensate polymer, or a meta-xyloyldiamine-adipic acid condensate polymer and a soft segment of a polymer such as polyester or polyether; polymer alloys of the polyamide above and a high-flexibility resin (polymer blends, graft polymers, random polymers, etc.); the polyamides above softened for example with a plasticizer; and the mixtures thereof.
The polyester elastomer is a concept including block copolymers of a saturated polyester such as polyethylene terephthalate or polybutylene terephthalate with polyether or polyester, and additionally, the polymer alloys thereof, the saturated polyesters softened for example with a plasticizer, and the mixture thereof.
The material favorably used is preferably a polyamide elastomer, from the viewpoints of its processability and flexibility, and a typical example thereof is PEBAX manufactured by Elf atochem.

Then, as shown in Figure 18, a shrink tube 24 that shrinks in its diameter under heat is placed on the entire external surface of the external-layer tube 16 in process E. The material for the shrink tube 24 is preferably polytetrafluoroethylene, a perfluoroethylene-propene copolymer, or the like.

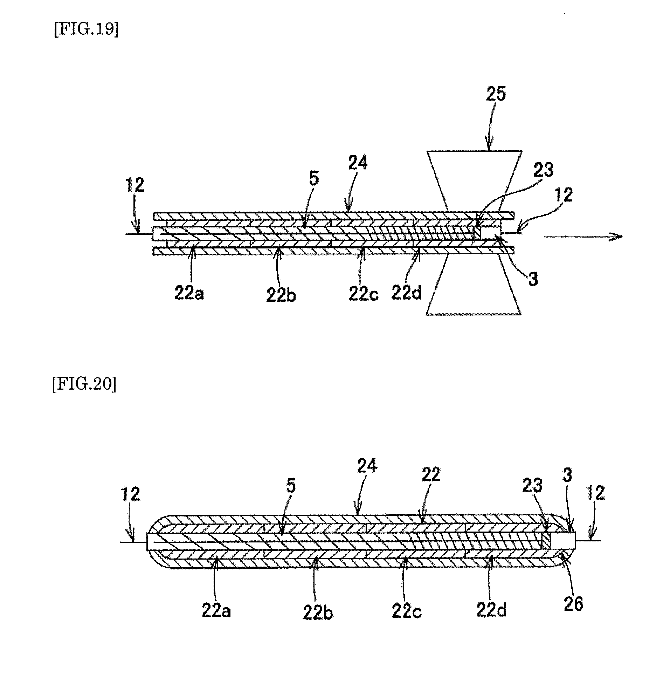

Then, the internal layer tube 3, the reinforcing-material layer 5, and the external-layer tube 22 are integrated, while the shrink tube 24 is heated to a tube-contracting temperature by a heater or application of high-frequency electromagnetic wave. The integration means that the internal layer tube 3, the reinforcing-material layer 5, and the external-layer tube 22 are bound to each other, restricting the mutual movement. As shown in Figure 19, the entire catheter covered with the shrink tube 24 may be sent through a circular hole in a heated mold 25 for ensuring integration.

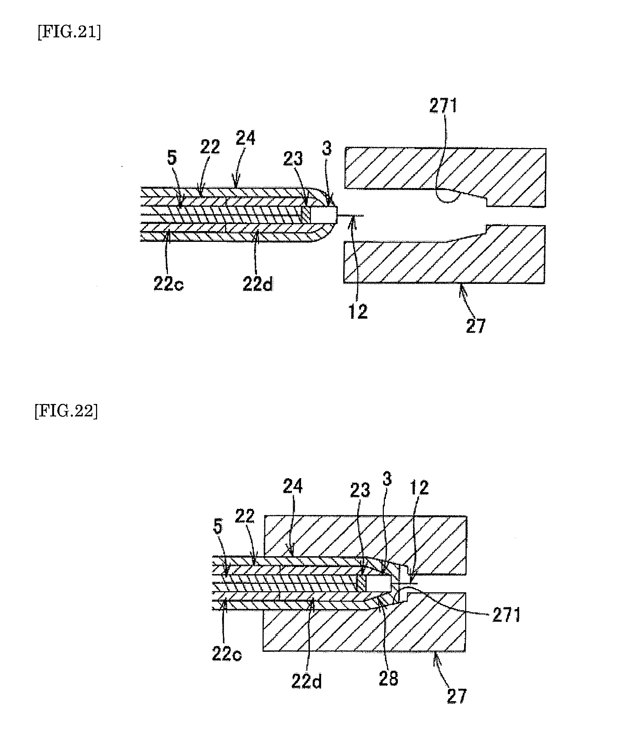

Contraction of the shrink tube 24 results in conversion of the distal region of the external-layer tube 22 into a rounded shape 26, as shown in Figure 20. In converting the distal region of the external-layer tube 22 into a tapered shape, the shrink tube 24 is first contracted, and, then as shown in Figure 21, the distal region of the resin tube is converted into a tapered shape 28 as it is brought into contact with a heated mold 27 shown in Figure 22 having a desirable tapered shape 271 on the internal surface.

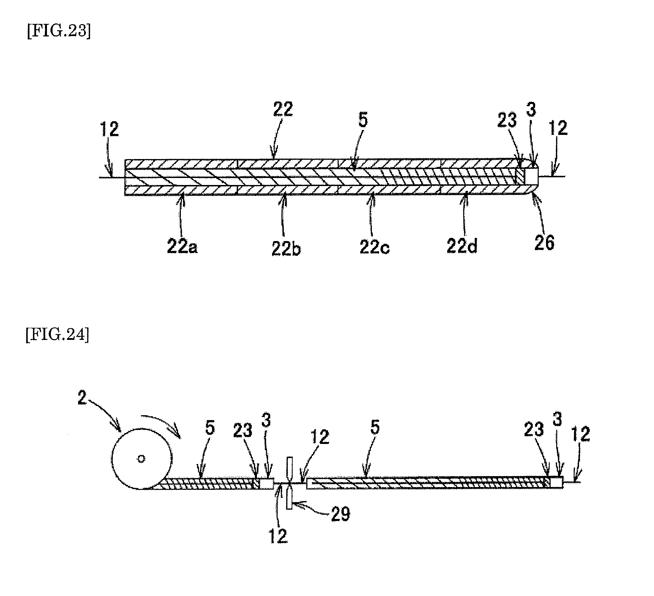

The shrink tube 24 is then separated as shown in Figure 23, and the internal layer tube 3, the reinforcing-material layer 5, and the external-layer tube 22 in the distal and proximal terminals of the catheter are cut off or adjusted as needed.
These are processings performed in the process E.

The process F is a step of connecting the catheters cut off in the process A into a continuous member, while the metal core wires 12 are welded. Welding is performed, for example, in a spot welding machine 29 as shown in Figure 24, wherein the metal core wires 12 are butt-welded, and the product is wound again around the reel 2.
These are processings performed in the process F.

The process G is a step of coating the external-layer tube 22 continuously on the long-connected catheter after process B or F by switched extrusion; and the internal layer tube 3, the reinforcing-material layer 5, and the external-layer tube 22 are integrated, while the external-layer tube 22 is formed by coating extrusion in such a manner that the Shore D hardness changes in one or more steps, or while the external-layer tube 22 is formed by coating extrusion in such a manner that the Shore D hardness decreases gradually in the direction from the proximal region to the distal region when the Shore D hardness changes in multiple steps.

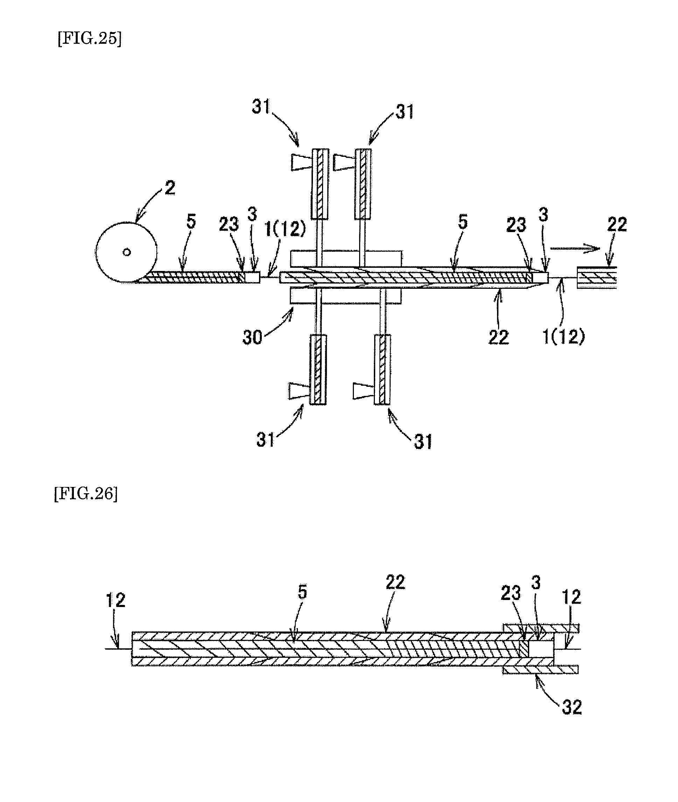

Then, when multiple resins, for example four resins, having different Shore D hardnesses are to be coated, the external-layer tube 22 is formed by feeding the four resins from four extruders 31 that are connected to one extrusion die 30, and adjusting the flow thereof to make the catheter have a desirable external diameter by operation and termination of the four extruders 31, as shown in Figure 25. Although not shown in the Figure, the external-layer tube 22 may be formed by continuously extruding the resins different in Shore D hardness by four extruders 31 connected to a die having a valve mechanism, one by one into the extrusion channel by switching operation and termination of the extruders. When there are multiple regions different in Shore D hardness, it is possible to adjust a rigidity/flexibility balance in a wider range by changing the winding pitch of the wire and also adjusting the length of the each region different in Shore D hardness. Although not shown in the Figure, when the X-ray-impermeable metal wire marker 19 or the X-ray-impermeable metal sheet marker 15 is not used, a marker may be formed in the most distal region of the external-layer tube 22 with a resin containing a blended X-ray-impermeable metal powder.

Then, the catheter is cut one by one; the terminal of the internal layer tube 3 or the external-layer tube 22 in the distal region is adjusted; and a shrink tube 32 that shrinks in its diameter under heat is placed only at the distal end, as shown in Figure 26. The material for a shrink tube 32 is preferably polytetrafluoroethylene, a perfluoroethylene -propene copolymer, or the like.

Similarly to the process E above, the internal layer tube 3, the reinforcing-material layer 5, and the external-layer tube 22 are integrated in the following step, while the shrink tube 32 is heated to a tube-contracting temperature by a heater or application of high-frequency electromagnetic wave. The entire catheter covered with the shrink tube 32 may then be sent through a die for ensuring integration. Then as shown in Figure 20 the distal region of the external-layer resin tube 22 is converted into the rounded shape 26 by contraction of the shrink tube 32. In forming the distal region of the external-layer resin tube 22 in the tapered shape, the shrink tube 32 is first contracted and converted into the tapered shape, as it is brought into contact with a heated mold 27 in a way similarly to Figure 21. The shrink tube 32 is removed after the conversion.
These are processings preformed in the process G.

Although not shown in the Figure, the catheter tube surface is preferably covered with a hydrophilic (or water-soluble) polymer substance, and thus, the composite is coated for improvement in hydrophilicity, subsequently in the process H. In this way, it is possible to reduce the friction coefficient of the catheter, making it more lubricant, to increase the lubricity of the catheter tube further even when the outermost surface of the catheter tube becomes in contact with blood, physiological saline, or the like, and consequently, to improve insertion efficiency, compatibility, kink resistance, and stability further. Examples of the hydrophilic polymer substances include the following natural or synthetic polymer substances and the derivatives thereof. Particularly favorable are cellulosic polymer substances (such as hydroxypropylcellulose), polyethylene oxide-based polymer substances (such as polyethylene glycol), maleic anhydride-based polymer substances (maleic anhydride copolymers such as methylvinylether-maleic anhydride copolymer), acrylamide-based polymer substances (such as polyacrylamide), and water-soluble nylons, because these materials give a low friction coefficient consistently.

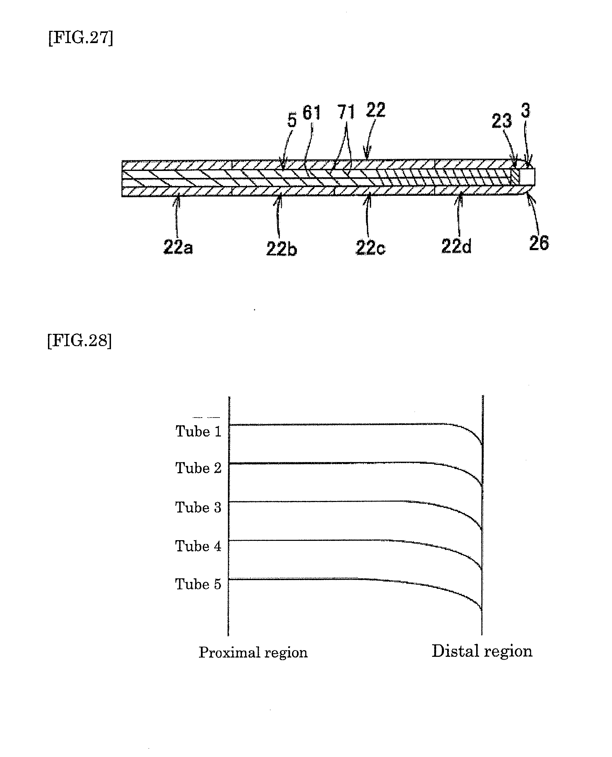

Further as shown in Figure 27, the metal core wire 1 is withdrawn, and the internal layer tube 3, the reinforcing-material layer 5, and the external-layer tube 22 at the proximal end are cleaved by means of a disk-shaped diamond cutter revolving at high speed, converting the proximal terminal cross section into a single plane and thus giving a catheter tube.

It is possible to make the rigidity/flexible inclination and the rigidity/flexible balance of the catheter tube suitable for various access routes more freely, by properly setting the braid pick distance, the length of the constant pick distance region, the order and the lengths of the resin tubes different in Shore D hardness. The rigidity/flexible balance is an indicator of the difference of the position of the high-flexibility region in the distal region or of the position of the change in bending strength, as shown in Figure 28. Figure 28 shows that the catheter in the straight region is higher in rigidity than that in the distal region, and yet retains a favorable flexibility as well. Proper adjustment and selection of the rigidity/flexible balance gives various advantages, and, for example, a catheter tube similar to the tube 1 shown in Figure 28 transmits the condition in distal region directly at high sensitivity and transmits torque efficiently, while a catheter tube similar to the tube 5 allows easier insertion into a deeper affected area via a complicated route and transmits the intention of surgeon's operational method to the affected part more effectively.

When the internal layer tube is formed with a fluorine resin such as polytetrafluoroethylene, the internal tube surface may be hydrophilized to a suitable degree by electrical means such as plasma discharge treatment.
In addition, although not shown in the Figure, it is possible to obtain a medical catheter tube in the most desirable shape by connecting a hub in a suitable shape to the proximal end in process H.
The medical catheter may be used as it is as described above or alternatively as it is bent as needed while a part of the medical catheter tube is heated by a heater or with steam.

## Claims

1. A medical catheter tube, comprising
an internal-layer tube of resin,
first and second reinforcing-material layers respectively of first and second wires formed on the internal-layer tube,
a marker of an X-ray impermeable metal formed covering the internal-layer tube, and
an external-layer tube of resin covering the internal-layer tube, the reinforcing-material layer, and the marker, wherein:
the catheter tube has a proximal region, a distal region, and a most distal region from the proximal terminal;
the first and second wires are made of a synthetic resin wire and/or a metal wire;
the first wire forms the first reinforcing-material layer formed in the catheter axial line direction;
the second wire forms the second reinforcing-material layer covering the first reinforcing-material layer as wound in the coil shape in the catheter circumferential direction;
the marker is formed in the distal region;
the marker is flexible to bending deformation; and
the flexural rigidity of the reinforcing-material layer and the external-layer tube decreases stepwise or continuously in the direction from the proximal to distal region.

2. The medical catheter tube according to Claim 1, wherein:
the resin tube of the internal-layer tube is lubricant and flexible;
the first and second wires provides the internal-layer tube with kink resistance, pressure resistance, torque-transmitting efficiency, and insertion efficiency;
the internal-layer tube, the reinforcing-material layer, the marker, and the external-layer tube are integrated;
the distal region is formed in the region distal from the reinforcing-material layer; and
the most distal region does not have the reinforcing-material layer or the marker.

3. The medical catheter tube according to Claim 1 or 2, wherein the marker is a coil of an X-ray impermeable metal wire wound in the coil shape around the internal-layer tube, a tube of an X-ray impermeable metal sheet having a slit and covering the internal-layer tube, or a tube formed with a resin containing a blended X-ray impermeable metal powder.

4. The medical catheter tube according to Claim 3, wherein the X-ray impermeable metal sheet is a square X-ray impermeable metal sheet having slits from both sides.

5. The medical catheter tube according to any one of Claims 1 to 4, wherein the first and second wires are made of a synthetic fiber having a thermoplastic liquid crystal polymer as its internal core and a flexible polymer as its sheath.

6. The medical catheter tube according to any one of Claims 1 to 5, wherein the winding pitch of the second wire of a synthetic resin wire and/or a metal wire forming the second reinforcing-material layer is changing continuously or stepwise in the direction from the proximal to distal region.

7. The medical catheter tube according to any one of Claims 1 to 6, wherein the external-layer tube has multiple segments; and the multiple segments are so aligned that the Shore D hardness of the multiple segments decreases stepwise in the direction from the proximal to distal region.

8. The medical catheter tube according to any one of Claims 1 to 7, wherein the internal- and external-layer tubes are connected to each other via the reinforcing-material layer and the marker.

9. The medical catheter tube according to any one of Claims 1 to 8, wherein the internal-layer tube is made of a resin lubricant to the guide wire and others extending therein.

10. The medical catheter tube according to any one of Claims 1 to 9, wherein the external-layer tube in the most distal region is molded into a rounded or tapered shape in which the external diameter thereof changes.

11. The medical catheter tube according to any one of Claims 1 to 10, wherein the external-layer tube is coated to be hydrophilic.

12. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the reinforcing-material layers are formed by placing a synthetic resin wire and/or a metal wire in the catheter axial line direction and winding a synthetic resin wire and/or a metal wire in the coil shape continuously, stepwise or at varying pitches in the catheter circumferential direction.

13. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering it with the external-layer tube, wherein: the external-layer tube is so formed on the internal-layer tube having the reinforcing-material layers and the marker that the Shore D hardness of the resin tubes thereof decreases stepwise in the direction from the proximal to distal region; the internal- and external-layer tubes are connected to each other via the reinforcing-material layer and the marker by heat shrinkage of the shrink tube on the external surface; and the shrink tube is removed after cooling.

14. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, changing the Shore D hardness of the resin regions for the external-layer tube stepwise in the axial direction, and adjusting the length of the resin regions different in Shore D hardness; the internal- and external-layer tubes are connected to each other via the reinforcing-material layer and the marker by heat shrinkage of the shrink tube on the external surface; and the shrink tube is removed after cooling.

15. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the external-layer tube is so extruded by switching extrusion method that the Shore D hardness decreases stepwise or continuously in the direction from the proximal to distal region on the internal-layer tube having the reinforcing-material layers and the marker formed; the internal- and external-layer tubes are connected to each other via the reinforcing-material layer and the marker by heat shrinkage of the shrink tube on the external surface; and the shrink tube is removed after cooling.

16. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, forming multiple resin regions different in Shore D hardness in the external-layer tube by switching extrusion method, and adjusting the length of the resin regions different in Shore D hardness; the internal- and external-layer tubes are connected to each other via the reinforcing-material layer and the marker by heat shrinkage of the shrink tube; and the shrink tube is removed after cooling.

17. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the external layer is extruded into multiple regions different in Shore D hardness formed on the internal-layer tube having the reinforcing-material layers and the marker by coat-switching extrusion molding in such a manner that the Shore D hardness decreases stepwise or continuously in the direction from the proximal to distal region; and the internal-layer tube, the reinforcing-material layer, the X-ray impermeable marker, and the external-layer tube are integrated.

18. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein a varying rigidity/flexibility balance is bestowed to the catheter by winding the synthetic resin wire and/or the metal wire continuously, stepwise, or at varying pitches in the coil shape in the catheter circumferential direction, extruding the external layer on the internal-layer tube having the reinforcing-material layers and the marker by coat-switching extrusion molding while changing the Shore D hardness thereof stepwise, adjusting the length of the resin regions different in shore D hardness; and the internal-layer tube, the reinforcing-material layer, the X-ray impermeable marker, and the external-layer tube are integrated.

19. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the X-ray impermeable marker is formed by winding an X-ray impermeable metal wire in the coil shape on the internal-layer tube in the region close to the distal side of the reinforcing-material layer, covering it with a square X-ray impermeable metal sheet having slits from both sides, or using a resin containing a blended X-ray impermeable metal powder, and the catheter is flexible in the distal region.

20. A method of producing the medical catheter tube according to any one of Claims 1 to 11, comprising forming the reinforcing-material layers on the external surface of the internal-layer tube, forming the X-ray impermeable marker flexible to bending deformation in the region to the distal side of the reinforcing-material layers, and covering them with the external-layer tube, wherein the most distal region of the external-layer tube is molded into a rounded or tapered shape.
